# EUROPEAN PATENT SPECIFICATION

(11) **EP 1 945 800 B1**
(45) Date of publication and mention of the grant of the patent: **14.03.2012**
(21) Application number: 06789292.7
(22) Date of filing: 02.08.2006
(51) Int. Cl.: C12Q 1/68

(54) **METHODS AND COMPOSITIONS FOR DETECTING AUTOIMMUNE DISORDERS**
VERFAHREN UND ZUSAMMENSETZUNGEN ZUM NACHWEIS VON AUTOIMMUNKRANKHEITEN
PROCÉDÉS DE COMPOSITIONS DE DÉTECTION DE TROUBLES AUTO-IMMUNS

(30) Priority: 05.08.2005 US 706205 P
(43) Date of publication of application: 23.07.2008
(60) Divisional application: 10188070.6
(73) Proprietor: Genentech, Inc., South San Francisco CA 94080-4990 (US)
(72) Inventor: ABBAS, Alexander, San Carlos, California 94070 (US)
(74) Representative: Walton, Seán Malcolm
(86) International application number: PCT/US2006/030241
(87) International publication number: WO 2007/019219

(56) References cited:
- WO-A-2006/020899
- WO-A2-03/090694
- WO-A2-2004/046098
- WO-A2-2004/047728
- US-A1- 2003 148 298
- US-A1- 2004 009 479
- US-A1- 2004 033 498
- US-A1- 2004 191 818
- "GeneChip Human Genome U133 Set" INTERNET CITATION, [Online] XP002232760 Retrieved from the Internet: URL:http://www.affymetrix.com/support/tech nical/datasheets/hgu133_datashe et.pdf> [retrieved on 2003-02-26]
- HAN ET AL: "ANALYSIS OF GENE EXPRESSION PROFILES IN HUMAN SYSTEMIC LUPUS ERYTHEMATOSUS USING OLIGONUCLEOTIDE MICROARRAY", GENES AND IMMUNITY, vol. 4, no. 3, 1 April 2003 (2003-04-01), pages 177-186, XP008029664, ISSN: 1466-4879
- CROW ET AL: "MICROARRAY ANALYSIS OF INTERFERON-REGULATED GENES IN SLE", AUTOIMMUNITY, vol. 36, no. 8, 1 December 2003 (2003-12-01), pages 481-490, XP008029657, ISSN: 0891-6934
- HAN ET AL: "ANALYSIS OF GENE EXPRESSION PROFILES IN HUMAN SYSTEMIC LUPUS ERYTHEMATOSUS USING OLIGONUCLEOTIDE MICROARRAY", GENES AND IMMUNITY, vol. 4, no. 3, 1 April 2003 (2003-04-01), pages 177-186, XP008029664, ISSN: 1466-4879
- CROW ET AL: "MICROARRAY ANALYSIS OF INTERFERON-REGULATED GENES IN SLE", AUTOIMMUNITY, vol. 36, no. 8, 1 December 2003 (2003-12-01), pages 481-490, XP008029657, ISSN: 0891-6934

## Description

### TECHNICAL FIELD

The present invention relates generally to the fields of molecular determination of autoimmune diseases. More specifically, the invention concerns methods and compositions based on unique molecular signatures associated with various aspects of autoimmune disorders.

### BACKGROUND

A number of autoimmune disorders are now believed to be characterized by the production of autoantibodies against a variety of self antigens. For example, systemic lupus erythematous (SLE) is an autoimmune disease in which autoantibodies cause organ damage by binding to host cells and tissues and by forming immune complexes that deposit in vascular tissues and activate immune cells. Sjogren's syndrome is an autoimmune disease characterized by inflammation in the glands of the body. Other autoimmune disorders are also commonly found, including but not limited to IgA nephropathy, psoriasis, rheumatoid arthritis, multiple sclerosis, ankylosing spondylitis, etc.

Interferon alpha (IFN-α) is a Type I interferon strongly implicated in the etiology of a number of immune disorders, such as SLE. It is believed that treatment approaches involving disruption of IFN-α signaling may be an effective treatment for such disorders. IFN-α levels are known to be elevated in SLE, and treatment of patients with IFN-α has been observed to reversibly cause symptoms similar to SLE in recipients. Numerous other lines of evidence have linked IFN-α and SLE.

The mechanisms by which IFN-α exerts its effects on the transcription of genes in target cells have been extensively investigated. The second messenger cascade has been determined, cis-regulatory binding sites for activated transcription factors have been defined, and several studies have explored what genes' expression is modulated. The most comprehensive of these studies have been performed with oligonucleotide microarrays, but definitions of interferon response gene expression profiles are still not complete because until recently microarrays have not contained a very complete set of reporters for the genes of the human genome.

One of the most difficult challenges in clinical management of autoimmune diseases is the accurate and early identification of the diseases in a patient. To this end, it would be highly advantageous to have molecular-based diagnostic methods that can be used to objectively identify presence and/or extent of disease in a patient. The invention described herein provides these methods and other benefits.

WO03090694 discloses marker sets for diagnosing or monitoring auto immune and chronic inflammatory diseases, particularly SLE.

### DISCLOSURE OF THE INVENTION

The invention relates to methods and compositions for identifying autoimmune disorders based at least in part on identification of the genes whose expression is associated with presence and/or extent of systemic lupus erythematosus (SLE), wherein SLE is in turn a prototypical autoimmune disease whose disease-associated gene signatures are also applicable in other autoimmune diseases. For example, as described herein, in one embodiment, genes modulated in response to signaling by IFN-α were identified. Information generated by this approach was then tested and modified to develop a concise and quantitative measure of the degree to which cell or tissue samples exhibit responses characteristic of autoimmune disorders. As shown herein, detection of one or more of specific genes disclosed herein can be a useful and informative indicator of presence and/or extent of autoimmune disorders in a patient. Moreoever, metrics or equivalent quotients that are indicative of interferon-associated disease presentation and/or severity can be generated by appropriate transformation of biomarker gene expression information. Exemplary transformations and resultant metrics are disclosed herein, generated based on gene expression data that are also disclosed herein.

In one aspect, the invention provides a method as set out in claim1.

In one aspect, the invention provides a method as set out in claim 2.

Disclosed herein is a method for monitoring minimal residual disease in a subject treated for an autoimmune disease, said method comprising determining whether the subject comprises a cell that expresses at least 2, 3, 4, 5, 6, 7, 8, 9, 10, 11, 12, 13, 14, 15, 16, 17, 18, 19, 20 or any number up to all of the genes (or genes associated with probesets) listed in Table 1, 2, 3, 4, 5, 6, 7(i), 7(ii) or 7(iii) at a level greater than the expression level of the respective genes in a normal reference sample, wherein detection of said cell is indicative of presence of minimal residual autoimmune disease.

Disclosed herein is a method for detecting an autoimmune disease state in a subject, said method comprising determining whether the subject comprises a cell that expresses at least 2, 3, 4, 5, 6, 7, 8, 9, 10, 11, 12, 13, 14, 15, 16, 17, 18, 19, 20 or any number up to all of the genes (or genes associated with probesets) listed in Table 1, 2, 3, 4, 5, 6, 7(i), 7(ii) or 7(iii) at a level greater than the expression level of the respective genes in a normal reference sample, wherein detection said cell is indicative of presence .of.an autoimmune disease state in the subject.

In one aspect, the invention provides a method as set out in claim 3.

Disclosed herein is a method for diagnosing an autoimmune disorder in a subject, said method comprising determining whether the subject comprises a cell that expresses at least 2, 3, 4, 5, 6, 7, 8, 9, 10, 11, 12, 13, 14, 15, 16, 17, 18, 19, 20 or any number up to all of the genes (or genes associated with probesets) listed in Table 1, 2, 3, 4, 5, 6, 7(i), 7(ii) or 7(iii) at a level greater than the expression level of the respective genes in a normal reference sample, wherein detection of said cell indicates that the subject has said autoimmune disorder.

The genes may be selected from the genes (or genes associated with the probesets) listed in Table 2, wherein the genes in Table 2 comprise a subgroup of the genes listed in Table 1. The selected genes may comprise at least 1, 2, 3, 4, 5, 6, 7 or all of the genes (or genes associated with probesets) listed in Table 2. The genes may also be selected from the genes (or genes associated with the probesets) listed in Table

Methods of the invention provide information useful for determining appropriate clinical intervention steps, if and as appropriate. Therefore, as disclosed herein the method may further comprises a clinical intervention step based on results of the assessment of the expression of one or more of the genes (or genes associated with probesets) listed in Table 1, 2, 3, 4, 5, 6 or 7. For example, appropriate intervention may involve prophylactic and treatment steps, or adjustment(s) of any then-current prophylactic or treatment steps based on gene expression information obtained by a method of the invention.

As would be evident to one skilled in the art, in any method of the invention, while detection of increased expression of a gene would positively indicate a characteristic of a disease (e.g., presence, stage or extent of a disease), non-detection of increased expression of a gene would also be informative by providing the reciprocal characterization of the disease.

Disclosed herein is an array/gene chip/gene set comprising polynucleotides capable of specifically hybridizing to at least 2, 3, 4, 5, 6, 7, 8, 9, 10,11,12, 13, 14, 15, 16, 17, 18, 19, 20 or all of the genes (or genes associated with probesets) listed in Table 1, and/or to at least 1, 2, 3, 4, 5, 6, 7 or all of the genes (or genes associated with probesets) listed in Table 2, and/or to at least 2 or any number up to all of the genes (or genes associated with probesets) listed in Table 3, 4, 5, 6 or 7. as set out in claim 5.

Disclosed herein is a kit comprising a composition, and instructions for using the composition to detect an autoimmune disorder by determining whether expression of at least 2, 3, 4, 5, 6, 7, 8, 9, 10, 11, 12, 13, 14, 15, 16, 17; 18, 19, 20 or all of the genes (or genes associated with probesets) listed in Table 1, and/or at least 1, 2, 3, 4, 5, 6, 7 or all of the genes (or genes associated with probesets) listed in Table 2, and/or at least 2 or any number up to all of the genes (or genes associated with probesets) listed in Table 3, 4, 5, 6 or 7 are at a level greater than the expression level of the respective genes in a normal reference sample. The composition may be an array/gene chip/gene set capable of specifically hybridizing to at least 2, 3, 4, 5, 6, 7, 8, 9, 10, 11, 12, 13, 14, 15, 16, 17, 18, 19, 20 or all of the genes (or genes associated with probesets) listed in Table 1, and/or to at least 1, 2, 3, 4, 5, 6, 7 or all of the genes (or genes associated with probesets) listed in Table 2, and/or to at least 2 or any number up to all of the genes (or genes associated with probesets) listed in Table 3, 4, 5, 6 or 7. The composition may comprise nucleic acid molecules encoding at least a portion of a polypeptide encoded by a gene (or gene associated with a probeset) listed in Table 1, 2, 3, 4, 5, 6 or 7. The composition may comprise a binding agent that specifically binds to at least a portion of a polypeptide encoded by a gene (or gene associated with a probeset) listed in Table 1, 2, 3, 4, 5, 6 or 7.

Methods and compositions disclosed herein may comprise one or more of the genes listed in Table 1, 2, 3, 4, 5, 6 or 7. If more than one gene is utilized or included , the more than one genes can be any combination of any number of the genes (or genes associated with probesets) as listed (in no particular order) in Table 1, 2, 3, 4, 5, 6 or 7. For example, a combination of genes comprises only two genes that correspond to the probesets as listed in Table 7(i). Disclosed herein is, a combination of genes comprises the genes associated with the probesets of Table 7(i), and one or more of the other genes (or genes associated with probesets) listed in Table 1, 2, 3, 4, 5 or 6. For example, one such combination may comprise genes associated with the probesets listed in Table 7(ii), and another such combination may comprise genes associated with the probesets listed in Table 7(iii). Disclosed herein is a combination of genes comprises one or more of the genes (or genes associated with probesets) listed in Table 1, 2, 3, 4, 5, 6 or 7, further combined with one or more other genes (or genes associated with probesets) that are not listed in Table 1, 2, 3, 4, 5, 6 or 7 (e.g., a gene known to be associated with an autoimmune disease but not associated with induction by interferons specifically).

Disclosed herein is a method of identifying a metric value correlated with presence and/or extent of an autoimmune disorder in a subject or sample, said method comprising:
(a) estimating a group of probesets that is collectively associated with a pattern
   wherein expression of genes represented by the probesets is associated with a disease characteristic;
(b) generating a weighting factor that weight probesets in accordance with a scale reflecting extent of match of each invidual probeset to trend of the group of probesets, and calculating the correlation coefficient of each probeset's profile to the mean profile calculated;
(c) determining a scaling factor, wherein the scaling factor is the value required to scale individual probesets to 1;
(d) multiplying the scaling factor by the weighting factor to generate a composite factor;
(e) multiplying a normal blood sample's signatures with the composite factor, and the averaging the resulting values across both probesets and samples to generate an average value, and inverting the average value to yield a global scaling factor;
(f) multiplying each weighting factor by the global scaling factor to obtain a vector of scalar values, and multiplying the scalar values by an expression signature from a sample of interest, and averaging the resulting values to yield a single metric that is indicative of degree of gene expression associated with Type I interferons in the sample.

In the method of the preceding paragraph, in step (a), the group of probesets may comprise probesets that include, or cluster around, the core most-tightly-correlated pair of probesets in subcluster associated with a disease characteristic.

In the method of the preceding paragraphs, in step (b), the factor is generated by transforming expression data of the group of probesets into z-scores comprising mean scaling to 1, base-2 log transformation, then scaling to a standard deviation of the mean of 1.

In the method of the preceding paragraphs, in step (e), the global scaling factor is useful for transforming output of the average of probesets from a sample of interest into a metric, wherein the metric is 1 if the sample is from a normal, healthy subject.

The group of probesets may comprise at least 2, 3, 4, 5, 6, 7, 8, 9, 10, 11, 12, 13, 14, 15, 16, 17, 18, 19, 20, 21, 22, 23 or all of those listed in Table 1, and/or at least 2, 3, 4, 5, 6, 7, 8 or all of those listed in Table 2, and/or at least 2 or any number up to all of those listed in Table 3, 4, 5, 6 or 7. The group of probesets may comprise all those listed in Table 1, 2, 3, 4, .5,6,or 7. The group of probesets may comprise at least 2 (or any integer up to all) of those listed in Table 3, Table 4, Table 5 or Table 6. The group of probesets may comprise all those listed in Table 7(i), 7(ii) or 7(iii).

Disclosed herein is a method comprising comparing a first metric obtained by a method described herein for a sample obtained from a subject of interest to a reference metric obtained from a reference (e.g., normal, healthy, non-diseased) sample, wherein a first metric that is higher than a reference metric indicates presence of an autoimmune disorder in the subject of interest.

Disclosed herein is a method of predicting responsiveness of a subject to autoimmune disease therapy, said method comprising comparing a first metric obtained by a method described herein for a sample obtained from the subject to a reference metric obtained from a reference (e.g., normal, healthy, non-diseased) sample, wherein a first metric that is higher than a reference metric indicates the subject would be responsive to the autoimmune disease therapy.

Disclosed herein is a method for monitoring minimal residual disease in a subject treated for an autoimmune disease, said method comprising comparing a first metric obtained by a method described herein for a sample obtained from the subject to a reference metric obtained from a reference (e.g., normal, healthy, non-diseased and/or untreated) sample, wherein a first metric that is higher than a reference metric is indicative of presence of minimal residual autoimmune disease.

Disclosed herein is a method for detecting an autoimmune disease state, said method comprising comparing a first metric obtained by a method described herein for a sample from a subject suspected of having the autoimmune disease state to a reference metric obtained from a reference (e.g., normal, healthy, non-diseased) sample, wherein a first metric that is higher than a reference metric is indicative of presence of the autoimmune disease state in the subject.

Disclosed herein is a method for assessing predisposition of a subject to develop an autoimmune disorder, said method comprising comparing a first metric obtained by a method described herein for a sample obtained from the subject to a reference metric obtained from a reference (e.g., normal, healthy, non-diseased) sample, wherein a first metric that is higher than a reference metric is indicative of a predisposition for the subject to develop the autoimmune disorder.

Disclosed herein is a method for diagnosing an autoimmune disorder in a subject, said method comprising comparing a first metric obtained by a method described herein for a sample obtained from the subject to a reference metric obtained from a reference (e.g., normal, healthy, non-diseased) sample, wherein a first metric that is higher than a reference metric indicates that the subject has said autoimmune disorder.

Disclosed herein is a method for distinguishing between active and inactive disease states (e.g., active and inactive SLE) in a subject, said method comprising comparing a first metric obtained by a method described herein for a sample obtained from the subject to a reference metric obtained from a reference (e.g., normal, healthy, non-diseased) sample, wherein a first metric that is higher than a reference metric indicates that the subject has the autoimmune disorder in its active state.

A reference metric may be obtained using a method described herein for a sample from a control sample (e.g., as obtained from a healthy and/or non-diseased and/or untreated tissue, cell and/or subject).

The steps in the methods for examining expression of one or more biomarkers may be conducted in a variety of assay formats, including assays detecting mRNA expression, enzymatic assays detecting presence of enzymatic activity, and immunohistochemistry assays. Optionally, the tissue or cell sample comprises disease tissue or cells.

Disclosed herein are methods of treating a disorder in a mammal, such as an immune related disorder, comprising steps of obtaining tissue or a cell sample from the mammal, examining the tissue or cells for expression (e.g., amount of expression) of one or more biomarkers, and upon determining said tissue or cell sample expresses said one or more biomarkers (e.g., wherein the biomarkers are expressed in amounts greater than a reference (control) sample), administering an effective amount of a therapeutic agent to said mammal. The steps in the methods for examining expression of one or more biomarkers may be conducted in a variety of assay formats, including assays detecting mRNA expression, enzymatic assays detecting presence of enzymatic activity, and immunohistochemistry assays. Optionally, the methods comprise treating an autoimmune disorder in a mammal. Optionally, the methods comprise administering an effective amount of a targeted therapeutic agent (e.g., an antibody that binds and/or block activity of Type 1 interferons and/or their corresponding receptor(s)), and a second therapeutic agent (e.g., steroids, etc.) to said mammal.

Biomarkers may be selected from those listed in Tables 1, 2, 3, 4, 5, 6 or 7.

### BRIEF DESCRIPTION OF THE DRAWINGS

FIG. 1 is a graphical depiction of analysis of IRGM data for ranked individual normal and SLE patient whole blood cell (WBC) samples.
FIG. 2 is a graphical depiction of analysis of IRGM data for ranked individual normal and IgA nephropathy patient whole blood cell (WBC) samples.
FIG. 3 is a graphical depiction of analysis of IRGM data for ranked individual normal, psoriatic lesional, and psoriatic non-lesional skin biopsy samples.
FIG. 4 is a graphical depiction of correlation of SLEDAI scores with IRGM.
FIG. 5 is a density plot showing a high concentration region of interferon-induced genes in a two dimensional cluster of whole-genome gene expression data from control and SLE whole blood samples.
FIG. 6 depicts distinct means of Type I Interferon Response Gene Metrics (IRGM) for SLE and healthy control patients.
FIG. 7 depicts distinct distributions of Type I Interferon Response Gene Metrics (IRGM) for SLE and healthy control patients.

### MODES FOR CARRYING OUT THE INVENTION

### General Techniques

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., eds., 1994).

Primers, oligonucleotides and polynucleotides employed in the present invention can be generated using standard techniques known in the art.

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### Definitions

The term "array" or "microarray", as used herein refers to an ordered arrangement of hybridizable array elements, preferably polynucleotide probes (e.g., oligonucleotides), on a substrate. The substrate can be a solid substrate, such as a glass slide, or a semi-solid substrate, such as nitrocellulose membrane. The nucleotide sequences can be DNA, RNA, or any permutations thereof.

A "target sequence", "target nucleic acid" or "target protein", as used herein, is a polynucleotide sequence of interest, in which a mutation is suspected or known to reside, the detection of which is desired. Generally, a "template," as used herein, is a polynucleotide that contains the target nucleotide sequence. In some instances, the terms "target sequence," "template DNA," "template polynucleotide," "target nucleic acid," "target polynucleotide," and variations thereof, are used interchangeably.

"Amplification," as used herein, generally refers to the process of producing multiple copies of a desired sequence. "Multiple copies" mean at least 2 copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

Expression/amount of a gene or biomarker in a first sample is at a level "greater than" the level in a second sample if the expression level/amount of the gene or biomarker in the first sample is at least about 1.5X, 1.75X, 2X, 3X, 4X, 5X, 6X, 7X, 8X, 9X or 10X the expression level/amount of the gene or biomarker in the second sample. Expression levels/amount can be determined based on any suitable criterion known in the art, including but not limited to mRNA, cDNA, proteins, protein fragments and/or gene copy. Expression levels/amounts can be determined qualitatively and/or quantitatively.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc. ), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α- anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

A "primer" is generally a short single stranded polynucleotide, generally with a free 3'-OH group, that binds to a target potentially present in a sample of interest by hybridizing with a target sequence, and thereafter promotes polymerization of a polynucleotide complementary to the target.

The phrase "gene amplification" refers to a process by which multiple copies of a gene or gene fragment are formed in a particular cell or cell line. The duplicated region (a stretch of amplified DNA) is often referred to as "amplicon." Usually, the amount of the messenger RNA (mRNA) produced, i.e., the level of gene expression, also increases in the proportion of the number of copies made of the particular gene expressed.

The term "mutation", as used herein, means a difference in the amino acid or nucleic acid sequence of a particular protein or nucleic acid (gene, RNA) relative to the wild-type protein or nucleic acid, respectively. A mutated protein or nucleic acid can be expressed from or found on one allele (heterozygous) or both alleles (homozygous) of a gene, and may be somatic or germ line.

To "inhibit" is to decrease or reduce an activity, function, and/or amount as compared to a reference.

The term "3"' generally refers to a region or position in a polynucleotide or oligonucleotide 3' (downstream) from another region or position in the same polynucleotide or oligonucleotide. The term "5"' generally refers to a region or position in a polynucleotide or oligonucleotide 5' (upstream) from another region or position in the same polynucleotide or oligonucleotide.

"Detection" includes any means of detecting, including direct and indirect detection.

The term "diagnosis" is used herein to refer to the identification of a molecular or pathological state, disease or condition, such as the identification of an autoimmune disorder. The term "prognosis" is used herein to refer to the prediction of the likelihood of autoimmune disorder-attributable disease symptoms, including, for example, recurrence, flaring, and drug resistance, of an autoimmune disease. The term "prediction" is used herein to refer to the likelihood that a patient will respond either favorably or unfavorably to a drug or set of drugs. In one embodiment, the prediction relates to the extent of those responses. In one embodiment, the prediction relates to whether and/or the probability that a patient will survive or improve following treatment, for example treatment with a particular therapeutic agent, and for a certain period of time without disease recurrence. The predictive methods of the invention can be used clinically to make treatment decisions by choosing the most appropriate treatment modalities for any particular patient. The predictive methods of the present invention are valuable tools in predicting if a patient is likely to respond favorably to a treatment regimen, such as a given therapeutic regimen, including for example, administration of a given therapeutic agent or combination, surgical intervention, steroid treatment, etc., or whether long-term survival of the patient, following a therapeutic regimen is likely.

The term "long-term" survival is used herein to refer to survival for at least 1 year, 5 years, 8 years, or 10 years following therapeutic treatment.

The term "increased resistance" to a particular therapeutic agent or treatment option, when used in accordance with the invention, means decreased response to a standard dose of the drug or to a standard treatment protocol.

The term "decreased sensitivity" to a particular therapeutic agent or treatment option, when used in accordance with the invention, means decreased response to a standard dose of the agent or to a standard treatment protocol, where decreased response can be compensated for (at least partially) by increasing the dose of agent, or the intensity of treatment.

"Patient response" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (i.e., reduction, slowing down or complete stopping) of disease cell infiltration into adjacent peripheral organs and/or tissues; (5) inhibition (i.e. reduction, slowing down or complete stopping) of disease spread; (6) decrease of auto-immune response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment.

The term "interferon inhibitor" as used herein refers to a molecule having the ability to inhibit a biological function of wild type or mutated. Type 1 interferon. Accordingly, the term "inhibitor" is defined in the context of the biological role of Type 1 interferon. In one embodiment, an interferon inhibitor referred to herein specifically inhibits cell signaling via the Type 1 interferon/interferon receptor pathway. For example, an interferon inhibitor may interact with (e.g. bind to) interferon alpha receptor, or with a Type 1 interferon which normally binds to interferon receptor. In one embodiment, an interferon inhibitor binds to the extracellular domain of interferon alpha receptor. In one embodiment, an interferon inhibitor binds to the intracellular domain of interferon alpha receptor. In one embodiment, an interferon inhibitor binds to Type 1 interferon. In one embodiment, the Type 1 interferon is an interferon alpha subtype. In one embodiment, the Type 1 interferon is not interferon beta. In one embodiment, the Type 1 interferon is not interferon omega. In one embodiment, interferon biological activity inhibited by an interferon inhibitor is associated with an immune disorder, such as an autoimmune disoder. An interferon inhibitor can be in any form, so long as it is capable of inhibiting interferon/receptor activity; inhibitors include antibodies (e.g., monoclonal antibodies as defined hereinbelow), small organic/inorganic molecules, antisense oligonucleotides, aptamers, inhibitory peptides/polypeptides, inhibitory RNAs (e.g., small interfering RNAs), combinations thereof, etc.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (e.g., full length or intact monoclonal antibodies), polyclonal antibodies, monovalent, multivalent antibodies, multispecific antibodies (e.g., bispecific antibodies so long as they exhibit the desired biological activity) and may also include certain antibody fragments (as described in greater detail herein). An antibody can be chimeric, human, humanized and/or affinity matured.

"Antibody fragments" comprise only a portion of an intact antibody, wherein the portion preferably retains at least one, preferably most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequence in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann, Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more CDRs/HVRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-793 (1992) describes affinity maturation by VH and VL domain shuffling. Random mutagenesis of CDR/HVR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immnunol. 154(7):3310-9 (1995); and Hawkins et al, J. Mol. Biol. 226:889-896 (1992).

The term "Fc region" is used to define the C-terminal region of an immunoglobulin heavy chain which may be generated by papain digestion of an intact antibody. The Fc region may be a native sequence Fc region or a variant Fc region. Although the boundaries of the Fc region of an immunoglobulin heavy chain might vary, the human IgG heavy chain Fc region is usually defined to stretch from an amino acid residue at about position Cys226, or from about position Pro230, to the carboxyl-terminus of the Fc region. The Fc region of an immunoglobulin generally comprises two constant domains, a CH2 domain and a CH3 domain, and optionally comprises a CH4 domain. By "Fc region chain" herein is meant one of the two polypeptide chains of an Fc region.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "blocking" antibody or an "antagonist" antibody is one which inhibits or reduces biological activity of the antigen it binds. Such blocking can occur by any means, e.g. by interfering with protein-protein interaction such as ligand binding to a receptor. In on embodiment, blocking antibodies or antagonist antibodies substantially or completely inhibit the biological activity of the antigen.

An "autoimmune disease" herein is a non-malignant disease or disorder arising from and directed against an individual's own tissues. The autoimmune diseases herein specifically exclude malignant or cancerous diseases or conditions, especially excluding B cell lymphoma, acute lymphoblastic leukemia (ALL), chronic lymphocytic leukemia (CLL), Hairy cell leukemia and chronic myeloblastic leukemia. Examples of autoimmune diseases or disorders include, but are not limited to, inflammatory responses such as inflammatory skin diseases including psoriasis and dermatitis (e.g. atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE) (including but not limited to lupus nephritis, cutaneous lupus); diabetes mellitus (e.g. Type I diabetes mellitus or insulin dependent diabetes mellitis); multiple sclerosis; Reynaud's syndrome; autoimmune thyroiditis; Hashimoto's thyroiditis; allergic encephalomyelitis; Sjogren's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia) ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

The term "sample", as used herein, refers to a composition that is obtained or derived from a subject of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis. In some embodiments, methods and compositions of the invention are useful in attempts to delay development of a disease or disorder.

An "effective amount" refers to an amount effective, at dosages and for periods of time necessary, to achieve the desired therapeutic or prophylactic result. A "therapeutically effective amount" of a therapeutic agent may vary according to factors such as the disease state, age, sex, and weight of the individual, and the ability of the antibody to elicit a desired response in the individual. A therapeutically effective amount is also one in which any toxic or detrimental effects of the therapeutic agent are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

As used herein, the terms "type I interferon" and "human type I interferon" are defined as all species of native human and synthetic interferon which fall within the human and synthetic interferon-α, interferon and interferon-β classes and which bind to a common cellular receptor. Natural human interferon-α comprises 23 or more closely related proteins encoded by distinct genes with a high degree of structural homology (Weissmann and Weber, Prog. Nucl. Acid. Res. Mol. Biol., 33: 251 (1986); J. Interferon Res., 13: 443-444 (1993)). The human IFN-α locus comprises two subfamilies. The first subfamily consists of at least 14 functional, non-allelic genes, including genes encoding IFN-αA (IFN-α2), IFN-αB (IFN-α8), IFN-αC (IFN-α10), IFN-αD (IFN-α1), IFN-αE (IFN-α22), IFN-αF (IFN-α21), IFN-αG (IFN-α5), IFN-α16, IFN-α17, IFN-α4, IFN-α6, IFN-α7, and IFN-αH (IFN-α14), and pseudogenes having at least 80% homology. The second subfamily, α_{Π} or ω, contains at least 5 pseudogenes and 1 functional gene (denoted herein as "IFN-α_{Π}1" or "IFN-ω") which exhibits 70% homology with the IFN-α genes (Weissmann and Weber (1986)). The human IFN-β is generally thought to be encoded by a single copy gene.

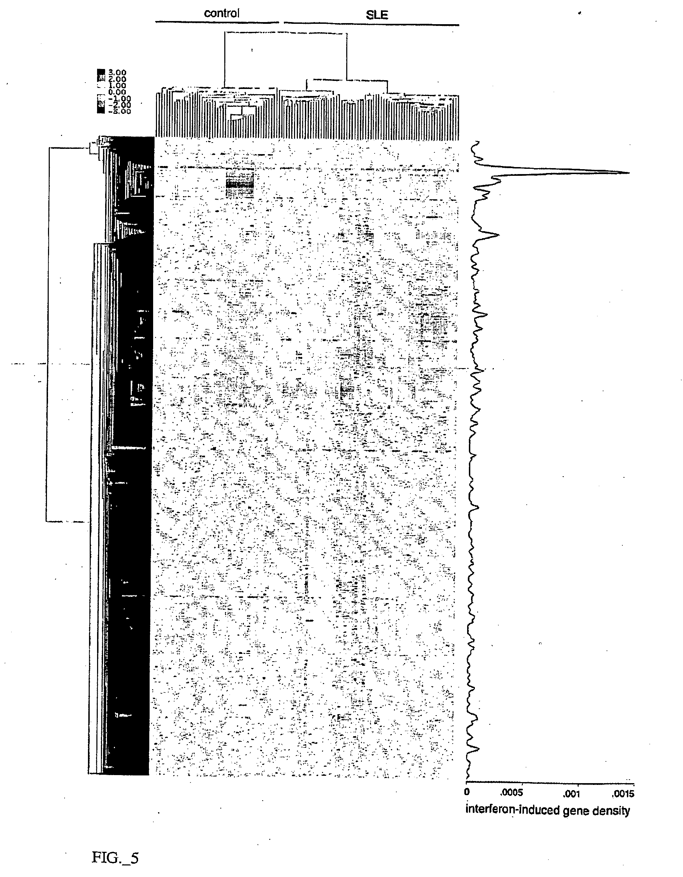

As used herein, the terms "first human interferon-α (hIFN-α) receptor", "IFN-αR", "hIFNAR1", "IFNAR1", and "Uze chain" are defined as the 557 amino acid receptor protein cloned by Uze et al., Cell, 60: 225-234 (1990), including an extracellular domain of 409 residues, a transmembrane domain of 21 residues, and an intracellular domain of 100 residues, as shown in Fig. 5 on page 229 of Uze *et al.* In one embodiment, the foregoing terms include fragments of IFNAR1 that contain the extracellular domain (ECD) (or fragments of the ECD) of IFNAR1.

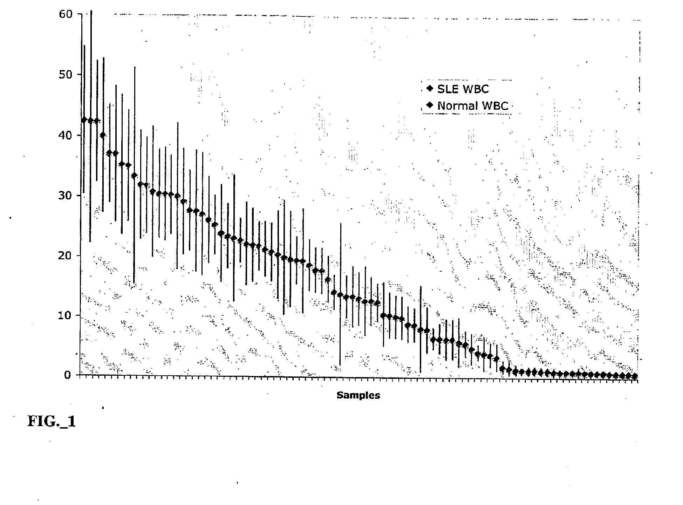

As used herein, the terms "second human interferon-α (hIFN-α) receptor", "IFN-αβR", "hIFNAR2", "IFNAR2", and "Novick chain" are defined as the 515 amino acid receptor protein cloned by Domanski et al., J. Biol. Chem., 37: 21606-21611 (1995), including an extracellular domain of 217 residues, a transmembrane domain of 21 residues, and an intracellular domain of 250 residues, as shown in Fig. 1 on page 21608 of Domanski *et al.* In one embodiment, the foregoing terms include fragments of IFNAR2 that contain the extracellular domain (ECD) (or fragments of the ECD) of IFNAR2, and soluble forms of IFNAR2, such as IFNAR2 ECD fused to at least a portion of an immunoglobulin sequence.

The term "housekeeping gene" refers to a group of genes that codes for proteins whose activities are essential for the maintenance of cell function. These genes are typically similarly expressed in all cell types. Housekeeping genes include, without limitation, glyceraldehyde-3- phosphate dehydrogenase (GAPDH), Cypl, albumin, actins, e.g. β-actin, tubulins, cyclophilin, hypoxantine phsophoribosyltransferase (HRPT), L32. 28S, and 185.

The term "biomarker" as used herein refers generally to a molecule, including a gene, protein, carbohydrate structure, or glycolipid, the expression of which in or on a mammalian tissue or cell can be detected by standard methods (or methods disclosed herein) and is predictive, diagnostic and/or prognostic for a mammalian cell's or tissue's sensitivity to treatment regimes based on inhibition of interferons, e.g. Type 1 interferons. Optionally, the expression of such a biomarker is determined to be higher than that observed for a control/reference tissue or cell sample. Optionally, for example, the expression of such a biomarker will be determined in a PCR or FACS assay to be at least 50-fold, or preferably at least 100- fold higher in the test tissue or cell sample than that observed for a control tissue or cell sample. Optionally, the expression of such a biomarker will be determined in an IHC assay to score at least 2 or higher for staining intensity. Optionally, the expression of such a biomarker will be determined using a gene chip-based assay.

An "IRG" or "interferon response gene", as used herein, refers to one or more of the genes, and corresponding gene products, listed in Tables 1 and 2. As shown herein, aberrant expression levels/amounts of one or more of these genes are correlated with a variety of autoimmune disorders. As would be evident to one skilled in the art, depending on context, the term IRG can refer to nucleic acid (e.g., genes) or polypeptides (e.g., proteins) having the designation or unique identifier listed in Tables 1, 2, 3, 4, 5, 6, and/or 7.

By "tissue or cell sample" is meant a collection of similar cells obtained from a tissue of a subject or patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like.

For the purposes herein a "section" of a tissue sample is meant a single part or piece of a tissue sample, e.g. a thin slice of tissue or cells cut from a tissue sample. It is understood that multiple sections of tissue samples may be taken and subjected to analysis according to the present invention, provided that it is understood that the present invention comprises a method whereby the same section of tissue sample is analyzed at both morphological and molecular levels, or is analyzed with respect to both protein and nucleic acid.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of gene expression analysis or protocol, one may use the results of the gene expression analysis or protocol to determine whether a specific therapeutic regimen should be performed.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable (*e.g*., radioisotope labels or fluorescent labels) or, in the case of an enzymatic label, may catalyze chemical alteration of a substrate compound or composition which is detectable.

### General illustrative techniques

A sample comprising a target molecule can be obtained by methods well known in the art, and that are appropriate for the particular type and location of the disease of interest. Tissue biopsy is often used to obtain a representative piece of disease tissue. Alternatively, cells can be obtained indirectly in the form of tissues/fluids that are known or thought to contain the disease cells of interest. For instance, samples of disease lesions may be obtained by resection, bronchoscopy, fine needle aspiration, bronchial brushings, or from sputum, pleural fluid or blood. Genes or gene products can be detected from disease tissue or from other body samples such as urine, sputum or serum. The same techniques discussed above for detection of target genes or gene products in disease samples can be applied to other body samples. Disease cells are sloughed off from disease lesions and appear in such body samples. By screening such body samples, a simple early diagnosis can be achieved for these diseases. In addition, the progress of therapy can be monitored more easily by testing such body samples for target genes or gene products.

Methods disclosed herein are useful for detecting any autoimmune disorder with which abnormal activation (e.g., overexpression) of interferons, in particular Type 1 interferons and/or their associated signaling pathway,is associated. The diagnostic methods of the present invention are useful for clinicians so that they can decide upon an appropriate course of treatment. For example, a sample from a subject displaying a high level of expression of the genes or gene products disclosed herein might suggest a more aggressive therapeutic regimen than a sample exhibiting a comparatively lower level of expression. Methods of the invention can be utilized in a variety of settings, including for example in aiding in patient selection during the course of drug development, prediction of likelihood of success when treating an individual patient with a particular treatment regimen, in assessing disease progression, in monitoring treatment efficacy, in determining prognosis for individual patients, in assessing predisposition of an individual to develop a particular autoimmune disorder (e.g., systemic lupus erythematosus, Sjogren's syndrome), in differentiating disease staging, etc.

Means for enriching a tissue preparation for disease cells are known in the art. For example, the tissue may be isolated from paraffin or cryostat sections. Disease cells may also be separated from normal cells by flow cytometry or laser capture microdissection. These, as well as other techniques for separating disease from normal cells, are well known in the art. If the disease tissue is highly contaminated with normal cells, detection of signature gene expression profile may be more difficult, although techniques for minimizing contamination and/or false positive/negative results are known, some of which are described hereinbelow. For example, a sample may also be assessed for the presence of a biomarker (including a mutation) known to be associated with a disease cell of interest but not a corresponding normal cell, or vice versa.

Disclosed herein is a variety of compositions suitable for use in performing methods of the invention. For example, the invention provides arrays that can be used in such methods. Disclosed herein is an array comprising individual or collections of nucleic acid molecules useful for detecting mutations of the invention. For instance, an array may comprises a series of discretely placed individual nucleic acid oligonucleotides or sets of nucleic acid oligonucleotide combinations that are hybridizable to a sample comprising target nucleic acids, whereby such hybridization is indicative of presence or absence of a mutation.

Several techniques are well-known in the art for attaching nucleic acids to a solid substrate such as a glass slide. One method is to incorporate modified bases or analogs that contain a moiety that is capable of attachment to a solid substrate, such as an amine group, a derivative of an amine group or another group with a positive charge, into nucleic acid molecules that are synthesized. The synthesized product is then contacted with a solid substrate, such as a glass slide, which is coated with an aldehyde or another reactive group which will form a covalent link with the reactive group that is on the amplified product and become covalently attached to the glass slide. Other methods, such as those using amino propryl silican surface chemistry are also known in the art, as disclosed at http://www.cmt.coming.com and http://cmgm.stanford.edu/pbrownl.

Attachment of groups to oligonucleotides which could be later converted to reactive groups is also possible using methods known in the art. Any attachment to nucleotides of oligonucleotides will become part of oligonucleotide, which could then be attached to the solid surface of the microarray.

Amplified nucleic acids can be further modified, such as through cleavage into fragments or by attachment of detectable labels, prior to or following attachment to the solid substrate, as required and/or permitted by the techniques used.

### TYPICAL METHODS AND MATERIALS OF THE INVENTION

The methods and assays disclosed herein are directed to the examination of expression of one or more biomarkers in a mammalian tissue or cell sample, wherein the determination of that expression of one or more such biomarkers is predictive or indicative of whether the tissue or cell sample will be sensitive to treatment based on the use of interferon inhibitors. The methods and assays include those which examine expression of biomarkers such as one or more of those listed in Tables 1, 2, 3, 4, 5, 6, and/or 7.

As discussed above, there are some populations of diseased human cell types that are associated with abnormal expression of interferons such as the Type 1 interferons which is associated with various autoimmune disorders. It is therefore believed that the disclosed methods and assays can provide for convenient, efficient, and potentially cost-effective means to obtain data and information useful in assessing appropriate or effective therapies for treating patients. For example, a patient having been diagnosed with an immune related condition could have a biopsy performed to obtain a tissue or cell sample, and the sample could be examined by way of various *in vitro* assays to determine whether the patient's cells would be sensitive to a therapeutic agent such as an interferon inhibitor (e.g., an anti-interferon alpha antibody or an antibody to interferon alpha receptor).

Disclosed herein are methods for predicting the sensitivity of a mammalian tissue or cells sample (such as a cell associated with an autoimmune disorder) to an interferon inhibitor. In the methods, a mammalian tissue or cell sample is obtained and examined for expression of one or more biomarkers. The methods may be conducted in a variety of assay formats, including assays detecting mRNA expression, enzymatic assays detecting presence of enzymatic activity, and immunohistochemistry assays. Determination of expression of such biomarkers in said tissues or cells will be predictive that such tissues or cells will be sensitive to the interferon inhibitor therapy. Applicants surprisingly found that the expression of such particular biomarkers correlates closely with presence and/or extent of various autoimmune disorders.

As discussed below, expression of various biomarkers in a sample can be analyzed by a number of methodologies, many of which are known in the art and understood by the skilled artisan, including but not limited to, immunohistochemical and/or Western analysis, quantitative blood based assays (as for example Serum ELISA) (to examine, for example, levels of protein expression), biochemical enzymatic activity assays, in situ hybridization, Northern analysis and/or PCR analysis of mRNAs, as well as any one of the wide variety of assays that can be performed by gene and/or tissue array analysis. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis).

The protocols below relating to detection of particular biomarkers, such as those listed in Tables 1, 2, 3, 4, 5, 6, and/or 7, in a sample are provided for illustrative purposes.

Methods disclosed herein include protocols which examine or test for presence of IRG in a mammalian tissue or cell sample. A variety of methods for detecting IRG can be employed and include, for example, immunohistochemical analysis, immunoprecipitation, Western blot analysis, molecular binding assays, ELISA, ELIFA, fluorescence activated cell sorting (FACS) and the like. For example, an optional method of detecting the expression of IRG in a tissue or sample comprises contacting the sample with a IRG antibody, a IRG-reactive fragment thereof, or a recombinant protein containing an antigen binding region of a IRG antibody; and then detecting the binding of IRG protein in the sample.

The expression of IRG proteins in a sample may be examined using immunohistochemistry and staining protocols. Immunohistochemical staining of tissue sections has been shown to be a reliable method of assessing or detecting presence of proteins in a sample. Immunohistochemistry ("IHC") techniques utilize an antibody to probe and visualize cellular antigens *in situ,* generally by chromogenic or fluorescent methods.

For sample preparation, a tissue or cell sample from a mammal (typically a human patient) may be used. Examples of samples include, but are not limited to, tissue biopsy, blood, lung aspirate, sputum, lymph fluid, etc. The sample can be obtained by a variety of procedures known in the art including, but not limited to surgical excision, aspiration or biopsy. The tissue may be fresh or frozen. In one embodiment, the sample is fixed and embedded in paraffin or the like.

The tissue sample may be fixed (*i.e*. preserved) by conventional methodology (See *e.g*., "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C.). One of skill in the art will appreciate that the choice of a fixative is determined by the purpose for which the sample is to be histologically stained or otherwise analyzed. One of skill in the art will also appreciate that the length of fixation depends upon the size of the tissue sample and the fixative used. By way of example, neutral buffered formalin, Bouin's or paraformaldehyde, may be used to fix a sample.

Generally, the sample is first fixed and is then dehydrated through an ascending series of alcohols, infiltrated and embedded with paraffin or other sectioning media so that the tissue sample may be sectioned. Alternatively, one may section the tissue and fix the sections obtained. By way of example, the tissue sample may be embedded and processed in paraffin by conventional methodology (See *e.g*., "Manual of Histological Staining Method of the Armed Forces Institute of Pathology", *supra*). Examples of paraffin that may be used include, but are not limited to, Paraplast, Broloid, and Tissuemay. Once the tissue sample is embedded, the sample may be sectioned by a microtome or the like (See *e.g*., "Manual of Histological Staining Method of the Armed Forces Institute of Pathology", *supra*). By way of example for this procedure, sections may range from about three microns to about five microns in thickness. Once sectioned, the sections may be attached to slides by several standard methods. Examples of slide adhesives include, but are not limited to, silane, gelatin, poly-L-lysine and the like. By way of example, the paraffin embedded sections may be attached to positively charged slides and/or slides coated with poly-L-lysine.

If paraffin has been used as the embedding material, the tissue sections are generally deparaffinized and rehydrated to water. The tissue sections may be deparaffinized by several conventional standard methodologies. For example, xylenes and a gradually descending series of alcohols may be used (See *e.g*., "Manual of Histological Staining Method of the Armed Forces Institute of Pathology", *supra*). Alternatively, commercially available deparaffinizing non-organic agents such as Hemo-De7 (CMS, Houston, Texas) may be used.

Optionally, subsequent to the sample preparation, a tissue section may be analyzed using IHC. IHC may be performed in combination with additional techniques such as morphological staining and/or fluorescence *in-situ* hybridization. Two general methods of IHC are available; direct and indirect assays. According to the first assay, binding of antibody to the target antigen (e.g., an IRG) is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies-may react with different epitopes on the primary antibody.

The primary and/or secondary antibody used for immunohistochemistry typically will be labeled with a detectable moiety. Numerous labels are available which can be generally grouped into the following categories:
(a) Radioisotopes, such as ³⁵S, ¹⁴C, ¹²⁵I, ³H, and ¹³¹I. The antibody can be labeled with the radioisotope using the techniques described in Current Protocols in Immunology, Volumes 1 and 2, Coligen et al., Ed. Wiley-Interscience, New York, New York, Pubs. (1991) for example and radioactivity can be measured using scintillation counting.
(b) Colloidal gold particles.
(c) Fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above. The fluorescent labels can be conjugated to the antibody using the techniques disclosed in *Current Protocols in Immunology, supra,* for example. Fluorescence can be quantified using a fluorimeter.
(d) Various enzyme-substrate labels are available and U.S. Patent No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (*e.g*., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g*., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed. J. Langone & H. Van Vunakis), Academic press, New York, 73:147-166 (1981).

Examples of enzyme-substrate combinations include, for example:
(i) Horseradish peroxidase (HRPO) with hydrogen peroxidase as a substrate, wherein the hydrogen peroxidase -oxidizes a dye precursor (*e.g*., orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (*e.g*., p-nitrophenyl-β-D-galactosidase) or fluorogenic substrate (*e.g*., 4-methylumbelliferyl-β-D-galactosidase).

Numerous other enzyme-substrate combinations are available to those skilled in the art. For a general review of these, see U.S. Patent Nos. 4,275,149 and 4,318,980. Sometimes, the label is indirectly conjugated with the antibody. The skilled artisan will be aware of various techniques for achieving this. For example, the antibody can be conjugated with biotin and any of the four broad categories of labels mentioned above can be conjugated with avidin, or *vice versa.* Biotin binds selectively to avidin and thus, the label can be conjugated with the antibody in this indirect manner. Alternatively, to achieve indirect conjugation of the label with the antibody, the antibody is conjugated with a small hapten and one of the different types of labels mentioned above is conjugated with an anti-hapten antibody. Thus, indirect conjugation of the label with the antibody can be achieved.

Aside from the sample preparation procedures discussed above, further treatment of the tissue section prior to, during or following IHC may be desired. For example, epitope retrieval methods, such as heating the tissue sample in citrate buffer may be carried out (see, *e.g.,* Leong et al. Appl. Immunohistochem. 4(3):201 (1996)).

Following an optional blocking step, the tissue section is exposed to primary antibody for a sufficient period of time and under suitable conditions such that the primary antibody binds to the target protein antigen in the tissue sample. Appropriate conditions for achieving this can be determined by routine experimentation. The extent of binding of antibody to the sample is determined by using any one of the detectable labels discussed above. Preferably, the label is an enzymatic label (*e.g*. HRPO) which catalyzes a chemical alteration of the chromogenic substrate such as 3,3'-diaminobenzidine chromogen. Preferably the enzymatic label is conjugated to antibody which binds specifically to the primary antibody (*e.g*. the primary antibody is rabbit polyclonal antibody and secondary antibody is goat anti-rabbit antibody).

Optionally, the antibodies employed in the IHC analysis to detect expression of an IRG are antibodies generated to bind primarily to the IRG of interest. Optionally, the anti-IRG antibody is a monoclonal antibody. Anti-IRG antibodies are readily available in the art, including from various commercial sources, and can also be generated using routine skills -known-in the art.

Specimens thus prepared may be mounted and coverslipped. Slide evaluation is then determined, *e.g*. using a microscope, and staining intensity criteria, routinely used in the art, may be employed. As one exmple, staining intensity criteria may be evaluated as follows:

**TABLE A**

| **Staining Pattern** | **Score** |
|---|---|
| No staining is observed in cells. | 0 |
| Faint/barely perceptible staining is detected in more than 10% of the cells. | 1+ |
| Weak to moderate staining is observed in more than 10% of the cells. | 2+ |
| Moderate to strong staining is observed in more than 10% of the cells. | 3+ |

In alternative methods, the sample may be contacted with an antibody specific for said biomarker under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labelled antibody to a target biomarker.

Sandwich assays are among the most useful and commonly used assays. A number of variations of the sandwich assay technique exist, and all are intended to be encompassed by the present invention. Briefly, in a typical forward assay, an unlabelled antibody is immobilized on a solid substrate, and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labelled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40°C such as between 25° C and 32° C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, -galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

It is contemplated that the above described techniques may also be employed to detect expression of IRG.

Disclosed herein are protocols which examine the presence and/or expression of mRNAs, such as IRG mRNAs, in a tissue or cell sample. Methods for the evaluation of mRNAs in cells are well known and include, for example, hybridization assays using complementary DNA probes (such as *in situ* hybridization using labeled IRG riboprobes, Northern blot and related techniques) and various nucleic acid amplification assays (such as RT-PCR using complementary primers specific for IRG, and other amplification type detection methods, such as, for example, branched DNA, SISBA, TMA and the like).

Tissue or cell samples from mammals can be conveniently assayed for, e.g., IRG mRNAs using Northern, dot blot or PCR analysis. For example, RT-PCR assays such as quantitative PCR assays are well known in the art. Disclosed herein is a method for detecting an IRG mRNA in a biological sample comprising producing cDNA from the sample by reverse transcription using at least one primer; amplifying the cDNA so produced using an IRG polynucleotide as sense and antisense primers to amplify IRG cDNAs therein; and detecting the presence of the amplified IRG cDNA. In addition, such methods can include one or more steps that allow one to determine the levels of IRG mRNA in a biological sample (e.g. by simultaneously examining the levels a comparative control mRNA sequence of a "housekeeping" gene such as an actin family member). Optionally, the sequence of the amplified IRG cDNA can be determined.

Disclosed herein are IRG primers and primer pairs, which allow the specific amplification of the polynucleotides disclosed herein or of any specific parts thereof, and probes that selectively or specifically hybridize to nucleic acid molecules disclosed herein or to any part thereof. Probes may be labeled with a detectable marker, such as, for example, a radioisotope, fluorescent compound, bioluminescent compound, a chemiluminescent compound, metal chelator or enzyme. Such probes and primers can be used to detect the presence of IRG polynucleotides in a sample and as a means for detecting a cell expressing IRG proteins. As will be understood by the skilled artisan, a great many different primers and probes may be prepared based on the sequences provided in herein and used effectively to amplify, clone and/or determine the presence and/or levels of IRG mRNAs.

Disclosed herein are protocols which examine or detect mRNAs, such as IRG mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes that have potential to be expressed in certain disease states may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment. (see, e.g., WO 01/75166 published October 11, 2001; (See, for example, U.S. 5,700,637, U.S. Patent 5,445,934, and U.S. Patent 5,807,522, Lockart, Nature Biotechnology, 14:1675-1680 (1996); Cheung, V.G. et al., Nature Genetics 21(Suppl):15-19 (1999) for a discussion of array fabrication). DNA microarrays are miniature arrays containing gene fragments that are either synthesized directly onto or spotted onto glass or other substrates. Thousands of genes are usually represented in a single array. A typical microarray experiment involves the following steps: 1) preparation of fluorescently labeled target from RNA isolated from the sample, 2) hybridization of the labeled target to the microarray, 3) washing, staining, and scanning of the array, 4) analysis of the scanned image and 5) generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (in situ).

The Affymetrix GeneChip® system is a commerically available microarray system which comprises arrays fabricated by direct synthesis of oligonucleotides on a glass surface. Probe/Gene Arrays: Oligonucleotides, usually 25 mers, are directly synthesized onto a glass wafer by a combination of semiconductor-based photolithography and solid phase chemical synthesis technologies. Each array contains up to 400,000 different oligos and each oligo is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligo. The Microarray Suite software subtracts the hybridization intensities of the mismatch probes from those of the perfect match probes to determine the absolute or specific intensity value for each probe set. Probes are chosen based on current information from Genbank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

The expression of a selected biomarker may also be assessed by examining gene deletion or gene amplification. Gene deletion or amplification may be measured by any one of a wide variety of protocols known in the art, for example, by conventional Southern blotting, Northern blotting to quantitate the transcription of mRNA (Thomas, Proc. Natl. Acad. Sci. USA, 77:5201-5205 (1980)), dot blotting (DNA analysis), or in situ hybridization (e.g., FISH), using an appropriately labeled probe, cytogenetic methods or comparative genomic hybridization (CGH) using an appropriately labeled probe. By way of example, these methods may be employed to detect deletion or amplification of IRG genes.

Expression of a selected biomarker in a tissue or cell sample may also be examined by way of functional or activity-based assays. For instance, if the biomarker is an enzyme, one may conduct assays known in the art to determine or detect the presence of the given enzymatic activity in the tissue or cell sample.

In the methods disclosed herein, it is contemplated that the tissue or cell sample may also be examined for the expression of interferons such as Type 1 interferons, and/or activation of the Type 1 interferon signaling pathway, in the sample. Examining the tissue or cell sample for expression of Type 1 interferons and/or the corresponding receptor(s), and/or activation of the Type interferon signaling pathway, may give further information as to whether the tissue or cell sample will be sensitive to an interferon inhibitor. By way of example, the IHC techniques described above may be employed to detect the presence of one of more such molecules in the sample. It is contemplated that in methods in which a tissue or sample is being examined not only for the presence of IRG, but also for the presence of, e.g., Type 1 interferon, interferon receptor(s), separate slides may be prepared from the same tissue or sample, and each slide tested with a reagent specific for each specific biomarker or receptor. Alternatively, a single slide may be prepared from the tissue or cell sample, and antibodies directed to each biomarker or receptor may be used in connection with a multi-color staining protocol to allow visualization and detection of the respective biomarkers or receptors.

Subsequent to the determination that the tissue or cell sample expresses one or more of the biomarkers indicating the tissue or cell sample will be sensitive to treatment with interferon inhibitors, it is contemplated that an effective amount of the interferon inhibitor may be administered to the mammal to treat a disorder, such as autoimmune disorder which is afflicting the mammal. Diagnosis in mammals of the various pathological conditions described herein can be made by the skilled practitioner. Diagnostic techniques are available in the art which allow, e.g., for the diagnosis or detection of autoimmune related disease in a mammal.

An interferon inhibitor can be administered in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering interferon inhibitors may be determined empirically, and making such determinations is within the skill in the art. Single or multiple dosages may be employed. For example, an effective dosage or amount of interferon inhibitor used alone may range from about 1 µg/kg to about 100 mg/kg of body weight or more per day. Interspecies scaling of dosages can be performed in a manner known in the art, e.g., as disclosed in Mordenti et al., Pharmaceut.Res., 8:1351 (1991).

When *in vivo* administration of interferon inhibitor is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

It is contemplated that yet additional therapies may be employed in the methods. The one or more other therapies may include but are not limited to, administration of steroids and other standard of care regimens for the particular autoimmune disorder in question. It is contemplated that such other therapies may be employed as an agent separate from the interferon inhibitor.

For use in the applications described or suggested above, kits or articles of manufacture are also disclosed herein. Such kits may comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Such probe may be an antibody or polynucleotide specific for IRG gene or message, respectively. Where the kit utilizes nucleic acid hybridization .to detect the target nucleic acid, the kit may also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label.

The kit disclosed herein will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for either *in vivo* or *in vitro* use, such as those described above.

The kits disclosed herein have a number of embodiments. A typical embodiment is a kit comprising a container, a label on said container, and a composition contained within said container; wherein the composition includes a primary antibody that binds to a IRG polypeptide sequence, the label on said container indicates that the composition can be used to evaluate the presence of IRG proteins in at least one type of mammalian cell, and instructions for using the IRG antibody for evaluating the presence of IRG proteins in at least one type of mammalian cell. The kit can further comprise a set of instructions and materials for preparing a tissue sample and applying antibody and probe to the same section of a tissue sample. The kit may include both a primary and secondary antibody, wherein the secondary antibody is conjugated to a label, e.g., an enzymatic label.

Disclosed herein is a kit comprising a container, a label on said container, and a composition contained within said container; wherein the composition includes a polynucleotide that hybridizes to a complement of the IRG polynucleotide under stringent conditions, the label on said container indicates that the composition can be used to evaluate the presence of IRG in at least one type of mammalian cell, and instructions for using the IRG polynucleotide for evaluating the presence of IRG RNA or DNA in at least one type of mammalian cell.

Other optional components in the kit include one or more buffers (e.g., block buffer, wash buffer, substrate buffer, etc), other reagents such as substrate (e.g., chromogen) which is chemically altered by an enzymatic label, epitope retrieval solution, control samples (positive and/or negative controls), control slide(s) etc.

The following are examples of the methods and compositions of the invention. It is understood that various other embodiments may be practiced, given the general description provided above.

### EXAMPLES

### Example 1

### Materials and Methods

Expression of IFN-α responsive genes (IRG's) was analyzed in data from blood - peripheral blood mononuclear cells (PBMC) and white blood cells (WBC) from normal donors and SLE patients from two sources: a collaboration with Tim Richardson at The University Of Michigan, and Genelogic Corporation.

The Richardson data was obtained as follows: blood was collected from 25 SLE patients and 20 healthy donors. RNA was prepared from PBMC by standard Ficoll gradient centrifugation and hybridized to HGU133P Affymetrix chips. Raw data was processed by Affymetrix MAS5 to yield Signal.

The Genelogic SLE data was obtained as follows: blood was collected from 73 SLE patients and 64 healthy controls. Globin mRNA was removed by affinity purification and the remaining mRNA was hybridized to HGU133 A and B chips according to standard protocols. Raw data was processed by Affymetrix MAS5 to yield Signal.

Microarray data was clustered in two dimensions (samples and probesets) using the xcluster software program (pearson on log2 signal) on probesets with mean signal > 100 and coefficient of variability greater than 0.2. Cluster data was viewed with the Java Treeview software program. Numerical analysis was performed with Excel (Microsoft, Redmond, WA).

### Results and Analysis

Microarray analysis was performed on the Richardson SLE samples. Samples clearly clustered by disease category (SLE or normal), and this pattern was robust to variation in the parameters of probeset filtering (50 < signal < 200, 0.2 < CV < 0.6). Several different tightly grouped probeset subclusters showed obvious biological commonalities. For instance, one subcluster was highly enriched for genes known to be specific to B cells, another to neutrophils, another for antibodies, and another for IRG's. The IRG subcluster showed an interesting pattern with respect to samples: normal samples all showed low expression of IRG's, while SLE samples showed a wide range of expression that varied from normal-like to extremely high.

The expression profiles of probesets within a tight subcluster are very similar but not identical, and the variation between very similar profiles may be due in significant part to noise either from biological or technological sources. For instance, some genes are represented on the microarray by more than one probeset, and there are several pairs of probesets in the IRG subcluster area that represent the same gene's expression. In these cases, the probesets clustered near to each other, sometimes but not always immediately adjacent. Thus it appeared that a clear pattern was present and reflected in many probesets, and that the pattern might most clearly be identified by utilizing the data from several probesets in order to limit the effect of noise. Nonetheless, the genes that were identified could be used as genetic identifiers that correlate with presence of disease.

### Development of a metric that correlates with disease, and identification of individual genres that may constitute such metric

We went on to attempt to measure the pattern by calculating a single metric proportional to the Signal levels of the specific subgroup of probesets. For example, we describe this approach below with the IRG probesets. The pattern (the aggregate profile of IRG's) was first defined roughly by visually estimating a group of probesets that appeared in Treeview to be the set that contained the pattern more than any other pattern and more than mere noise. This group was the two-hundred probesets that include or cluster around the core most-tightly-correlated pair of probesets in the subcluster.

The expression data of this group was then transformed into z-scores (mean scaled to 1, base-2 log transformed, then scaled to a standard deviation of the mean of 1), and the correlation coefficient of each probeset's profile to the mean profile was calculated. These correlation coefficients were used as weighting factors to weight relatively heavily the probesets that showed the strongest match to the trend of the group, and to weight relatively lightly those that apparently were more affected by other inputs or noise.

The factors required to scale probesets to 1 were multiplied by the weighting factor, to produce a composite factor that could yield a normalized, weighted metric for one data point. The normal blood samples' signatures were multiplied by that factor, averaged across both probesets and samples, and this number was inverted to yield a global scaling factor that would transform the output of the average of probesets from a sample into a metric that would be expected to be 1 if normal. Each normalization/weighting factor was multiplied by this factor. The result is a vector of scalar values that are multiplied by a sample expression signature and averaged to yield the Type I Interferon Response Gene Metric (IRGM), a single metric measuring the level of IFN- a transcriptional response in a sample. The number of probesets that was used in this metric was fewer than the original set of two-hundred that originally helped to define it. Twenty-four probesets were typically used (Table 1), although sets of eight to one hundred were tested and performed well.

For purposes of validation, the IRGM was determined for sets of biological samples of similar tissue type (i.e. all whole blood, all skin biopsy, etc.) and drawn from multiple groups that included at least one disease group and one normal group. The samples analyzed contained none of the samples used to generate the IRGM parameters. The measured metrics for different sets were compared to each other by combining them into a single rank-ordered list and evaluating the degree to which samples of a particular group segregate to a particular part of the list.

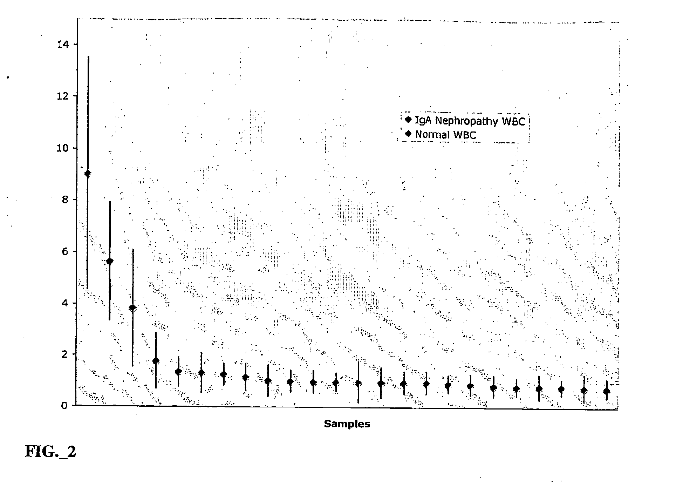

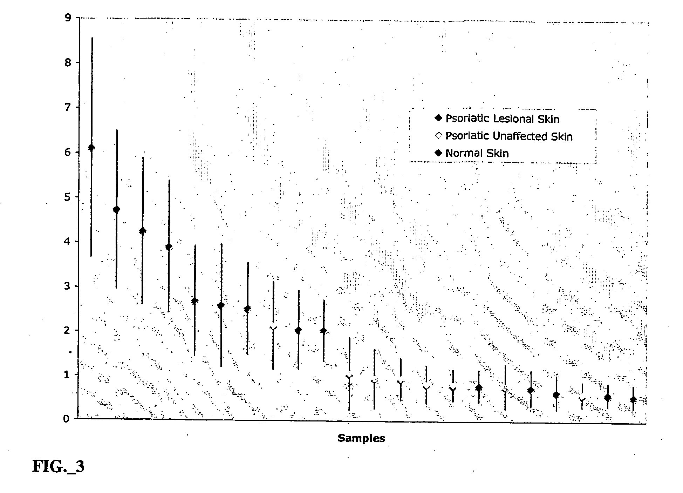

IRGM scores were calculated and evaluated for a set of samples completely distinct from those used for selection of the IRGM genes and training of the test paramters. Both PBMC and whole blood samples from healthy patients or patients suffering from SLE showed a very clear separation (Figure 1): healthy patients had relatively low IRGM, and tightly clustered, while SLE patients ranged fairly uniformly from the upper end of the normal range up to 40. Wegener's Disease, IgA nephropathy (Figure 2), rheumatoid arthritis all showed mild separation, with maximum disease sample scores between 5 and 10. Psoriasis blood samples showed similar patterns but less-marked separation, with maximum IRGM's around 7. Psoriatic skin biopsies were significantly high compared to both non-lesional skin biopsies from psoriasis patients and to skin biopsies from healthy donors (Figure 3).

For several genes in the IRGM probeset vector there is more than one probeset that represents them, giving an opportunity to gauge whether technical variation between probesets exerts a significant effect on the expression data observed. Members of each of these pairs of probesets were observed here to show expression profiles highly correlated to each other relative to the magnitude of their individual profiles and to cluster very closely together relative to probesets from other genes. This observation indicates that the data are accurate measurements of gene expression and that technical issues related to probe selection and probeset design have at most a minor negative effect.

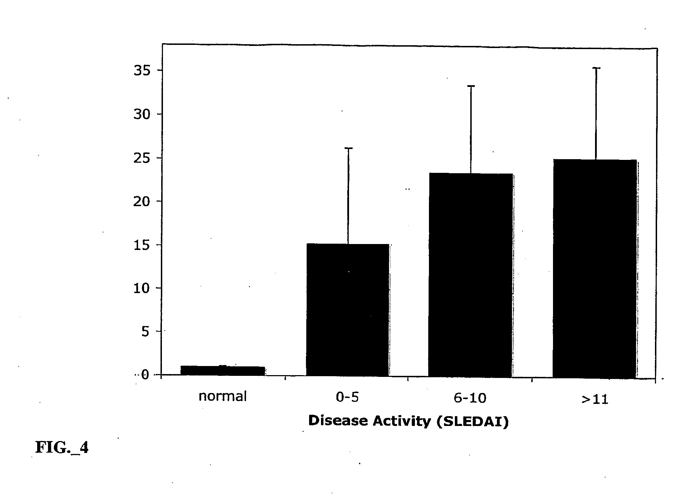

Clinical measures of SLE disease activity and severity such as SLEDAI quantitate patient disease symptoms and may correlate with expression of genes that underlie the etiology of the disease. In order to investigate this hypothesis, IRGM data on individual patients were compared to those patients' SLEDAI scores. Although overall the correlation appeared to be relatively weak (R = 0.2125), the correlation was statistically significant. Indeed, correlation was confirmed by the observation that the interaction was stronger when the SLEDAI scores were binned into three equal categories and the difference between the categories was tested (Figure 4).

The IRGM test, and expression of the genes that make up such a test (as set forth in Table 1), could be useful for selecting patients that would benefit from IFN-α-based treatment for autoimmune disorders (e.g., SLE) by identifying patients that have a relatively high IRGM score and thus have IFN-α signaling that could be blocked. Equivalently, it could be used to predict that certain patients would not benefit from IFN-α-based treatment because they do not exhibit a high IRGM score and thus are not currently experiencing active IFN-α signaling that could be disrupted.

The IRGM test, and expression of the genes that make up such a test (as set forth in Table 1), are useful indicators in a variety of drug development, diagnostic, prognostic and therapeutic settings as described above. For example, this information could be used to check whether patients that have responded well to anti-IFN-α treatment had high levels of expression of the signaling targets of IFN-α before treatment and afterwards whether the treatment abrogated that expression. It would be a useful gauge of the extent to which a particular treatment affects the IFN-α signaling pathway. It might be a useful bio- or pharmacodynamic marker, measuring the profile of the effects of treatment over time.

### Other Interferons

The metric-based approach described above could be utilized in a variety of ways in characterizing disease pathways, mechanisms of action and drug pharmacodynamics. For example, different interferon molecules probably have different properties that the IRGM and/or a test made the same way based on different microarray data and/or analyses could help measure and elucidate. For instance:
1) Type I interferons all signal through the same heterodimeric receptor but may differ in their half-life, receptor affinity, or power to initiate signaling in a target cell. These differences in magnitudes might be measured easily and accurately by IRGM. This sort of measurement could be carried out either in a cell culture experiment or in a clinical setting. Likewise, the effect of candidate drugs or drugs used in clinical settings can be gauged using this approach.
2) Different IRGM-like tests could be constructed by microarray assays of cultured blood samples treated with different interferons. To the extent to which the tests differ from each other, they could be applied to clinical samples to determine the relative activities of different interferons and/or drugs.

### Other Signatures

The method used to generate the IRGM test could also be applied to any sort of expression signature, either of a state or activity of cells or of a type of cell or cells. For instance, there are particular transcriptional changes associated with active mitotic cell replication. These transcriptional changes could be consolidated into a test that would be applied to a variety of biological samples to measure how actively they are dividing. Or in another example, the genes whose expression is specific to particular types of immune cells could be categorized by which cell type expresses them and then for each cell type a test could be made. This collection of tests could then be applied to any of a variety of clinical samples (blood from SLE patients, intestinal biopsies from Crohn's Disease patients, etc.) to determine the balance of immune cell types.

**Table 1. Probesets, unique database identifiers, and names corresponding to genes that have increased expression. These probesets were also used to generate a single metric test (also described as the IRGM test herein).**

| **probeset** | **Symbol** | **RefSeq ID (Symbols)** | **Name** |
|---|---|---|---|
| 226702_at | AFAR17068 | NM_207315 (THYK1) | similar to thymidylate kinase family LPS-inducible member |
| 223220_s_at | BAL | NM_031458 (PARP9) | B agressive lymphoma gene |
| 219863_at | ERRS16511 | NM_016323 (HERC5) | cyclin-E binding protein 1 (LOC51191) |
| 242625_at | CIG5 | NM_080657 (RSAD2) | Cig5 |
| 208436_s_at | IRF7 | NM_004029 (IRF7) | Interferon regulatory factor 7 (IRF7) |
| 204747_at | IFIT4 | NM_001549 (IFIT3) | Interferon induced -tetratricopeptide protein IFI60 (IFTT4) |
| 213797_at | CIG5 | NM_080657 (RSAD2) | Cig5 |
| 202086_at | MX1 | NM_002462 (MX1) | Myxovirus influenza resistance 1 |
| 213294_at | WTCF34654 | BG283489 (PRKR) | FLJ38348 |
| 227609_at | BRESI1 | NM_001002264 (EPSTI1) | Putative breast epithelial stromal interaction protein |
| 205483_s_at | Isg15 | NM_005101 (G1P2) | Interferon-stimulated protein (15 kDa) |
| 218943_s_at | RIG-1 | NM_014314 (DDX58) | AF038963 |
| 202446_s_at | P37 | NM_021105 (PLSCR1) | Phospholipid scramblase P37 |
| 214453_s_at | MTAP44 | NM_006417 (IFI44) | "Interferon-induced, hepatitis C-associated microtubular agg |
| 219356_s_at | HSPC177 | NM_016410 (SNF7DC2) | Chromatin modifying protein 5 |
| 203595_s_at | RI58 | NM_012420 (IFIT5) | Retinoic acid- and interferon-inducible protein (58kD) |
| 204439_at | VERC16692 | NM_006820 (IFI44L) | chromosome 1 open reading frame 29 |
| 218400_at | OAS3 | NM_006187 (OAS3) | 2'-5'-oligoadenylate synthetase 3 |
| 209762_x_at | Sp110 | NM_004509 (SP110) | Transcriptional coactivator Sp110 |
| 230036_at | SAMD9L | NM_152703 (C7orf6) | sterile alpha motif domain containing 9-like |
| 229450_at | IFIT4 | NM_001549 (IFIT3) | Interferon induced tetratricopeptide protein IFI60 (lFIT4) |
| 208966_x_at | ANNY16434 | NM_005531 (IFI16) | clone MGC:23885 IMAGE:4703266, mRNA, complete cds |
| 203153_at | IFIT1 | NM_001001887 (IFIT1) | Interferon-induced protein with tetratricopeptide repeats 1 |
| 226603_at | SAMD9L | NM_152703 (C7orf6) | sterile alpha motif domain containing 9-like |

**Table 2. One illustrative set of genes with increased expression in autoimmune disorders.**

| **probeset** | **Symbol** | **RefSeq identifier (Symbols)** | **Name** |
|---|---|---|---|
| 218400_at | OAS3 | NM_006187 (OAS3) | 2'-5'-oligoadenylate synthetase 3 |
| 218943_s_at | RIG-1 | NM_014314 (DDX58) | AF038963 |
| 219356_s_at | HSPC177 | NM_016410 (SNF7DC2) | Chromatin modifying protein 5 |
| 219863_at | ERRS16511 | NM_016323 (HERC5) | cyclin-E binding protein 1 (LOC51191) |
| 223220_s_at | BAL | NM_031458 (PARP9) | B agressive lymphoma gene |
| 226603_at | SAMD9L | NM_152703 (C7orf6) | sterile alpha motif domain containing 9-like |
| 226702_at | AFAR17068 | NM_207315 (THYK1) | similar to thymidylate kinase family LPS-inducible member |
| 227609_at | BRESI1 | NM_001002264 (EPSTI1) | Putative breast epithelial stromal interaction protein |
| 230036_at | SAMD9L | NM_152703 (C7orf6) | sterile alpha motif domain containing 9-like |

### Example 2

The approach used in Example 1 for defining probes and genes to be used as type I interferon signature markers was then extended to identify 78 probes (49 genes) as type I interferon signature markers and to illustrate their utility in diagnosing autoimmune disease patients (such as those with SLE) based on the expression levels of these genes in patient samples.

### Materials and Methods

Microarray data was obtained as follows: blood was collected from 76 SLE patients and 46 healthy controls. Globin mRNA was removed by affinity purification and the remaining mRNA was hybridized to HGU133 A and B chips according to standard protocols. Raw data was processed by the Affymetrix MAS5 algorithm to yield Signal data.

Microarray data was clustered in two dimensions (samples and probesets) using the xcluster software program (pearson on log2 signal) on probesets with mean signal > 100 and coefficient of variability greater than 0.2. Cluster data was viewed with the Java Treeview software program. Numerical analysis was performed with R. "R" is an open-source community-based project with the following characteristics: title = R: A Language and Environment for Statistical Computing; author = R Development Core Team; organization = R Foundation for Statistical Computing; address = Vienna, Austria; year = 2006; note = ISBN 3-900051-07-0.

### Results and Analysis

Type I interferon-induced genes were identified from Genbank annotation and various literature sources. These genes were mapped to Affymetrix probes and their density across a global cluster of SLE and healthy control whole blood cell microarray data was plotted with a bandwidth of 30 (Figure 5). The density curve of these probes revealed a broad but sparse distribution with a single very dense cluster of probes within the region of the cluster that defined the clear sample separation between SLE and healthy control samples by their marked upmodulation in SLE samples. Probes at the peak of the very dense region were relatively highly correlated in their expression patterns. The set of probes optimally diagnosing the type I interferon induction signature in SLE was defined as those containing the peak of the density curve and including all probes that were linked with a correlation coefficient of greater than 0.9 (Table 3).

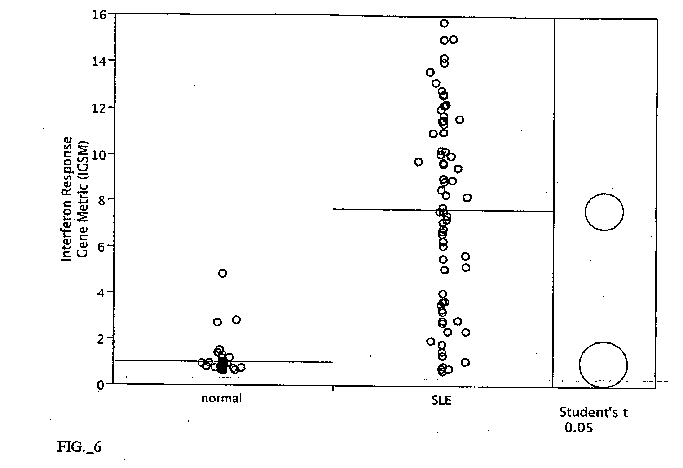

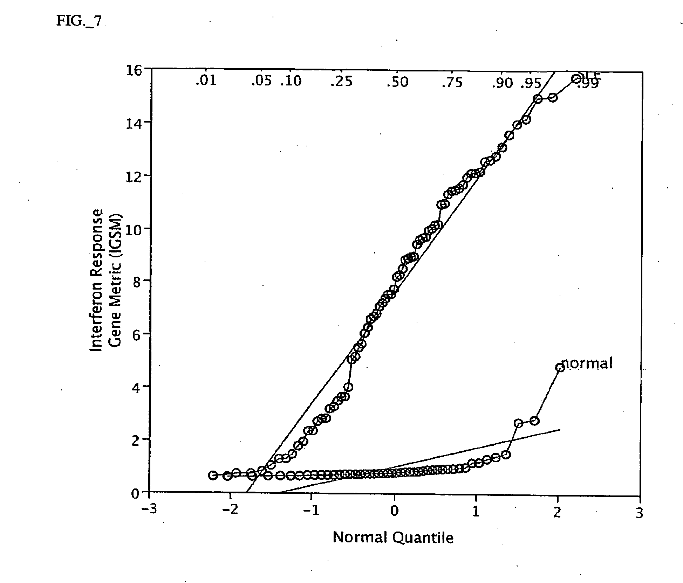

The Type I Interferon Response Gene Metric (IRGM) was calculated for each blood sample as previously described but based on the set of genes presented in Table 3. A Student's T Test shows the large (> 6-fold) and significant (p-value < .0001) difference between the mean of the two groups (Figure 6). A plot of distributions of the two groups of samples against normal quantiles shows differences in their distributions (Figure 7). The distribution of the control samples appears to be very low and log-normal with a few upper outliers, while the SLE samples are more equally (linearly) distributed across a large range from within the range of healthy controls up to very high levels. This large spread of IRGM scores could support a robust diagnostic for different categories of SLE disease state or type.

**Table 3. Probesets, unique database identifiers, symbols, and names corresponding to genes that show a pattern of interferon-induced expression in SLE and healthy control samples.**

| **Probe** | **Database Accession** | **Symbol** | **Name** |
|---|---|---|---|
| 213797_at | NM_080657 | RSAD2 | radical S-adenosyl methionine domain containing 2 |
| 226702_at | NM_207315 | LOC129607 | hypothetical protein LOC129607 |
| 214453_s_at | NM_006417 | IFI44 | interferon-induced protein 44 |
| 227609_at | NM_001002264 | EPSTI1 | epithelial stromal interaction 1 |
| 242625_at | NM_080657 | RSAD2 | radical S-adenosyl methionine domain containing 2 |
| 230036_at | NM_152703 | SAMD9L | sterile alpha motif domain containing 9-like |
| 214059_at | NM_006417 | IFI44 | interferon-induced protein 44 |
| 218400_at | NM_006187 | OAS3 | 2'-5'-oligoadenylate synthetase 3, 100kDa |
| 226603_at | NM_152703 | SAMD9L | sterile alpha motif domain containing 9-like |
| 219863_at | NM_016323 | HERC5 | hect domain and RLD 5 |
| 204439_at | NM_006820 | IFI44L | interferon-induced protein 44-like |
| 228617_at | NM_017523 | XIAPAF1 | XIAP associated factor-1, transcript variant 1 |
| 203596_s_at | NM_012420 | IFIT5 | interferon-induced protein with tetratricopeptide repeats |
| 204972_at | NM_001032731 | OAS2 | 2'-5'-oligoadenylate synthetase 2, 69/71kDa |
| 205483_s_at | NM_005101 | G1P2 | interferon, alpha-inducible protein (clone IFI-15K) |
| 219211_at | NM_017414 | USP18 | ubiquitin specific protease 18 |
| 223220_s_at | NM_031458 | PARP9 | poly (ADP-ribose) polymerase family, member 9 |
| 205660_at | NM_003733 | OASL | 2'-5'-oligoadenylate synthetase-like |
| 204747_at | NM_001031683 | IFIT3 | interferon-induced protein with tetratricopeptide repeats |
| 218943_s_at | NM_014314 | DDX58 | DEAD (Asp-Glu-Ala-Asp) box polypeptide 58 |
| 203153_at | NM_001001887 | IFIT1 | interferon-induced protein with tetratricopeptide repeats |
| 205552_s_at | NM_001032409 | OAS 1 | 2',5'-oligoadenylate synthetase 1, 40/46kDa |
| 224701_at | NM_017554 | PARP14 | poly (ADP-ribose) polymerase family, member 14 |
| 208436_s_at | NM_001572 | IRF7 | interferon regulatory factor 7 |
| 210797_s_at | NM_003733 | OASL | 2'-5'-oligoadenylate synthetase-like |
| 203595_s_at | NM_012420 | IFIT5 | interferon-induced protein with tetratricopeptide repeats |
| 219062_s_at | NM_017742 | ZCCHC2 | zinc finger, CCHC domain containing 2 |
| 202145_at | NM_002346 | LY6E | lymphocyte antigen 6 complex, locus E |
| 209417_s_at | NM_005533 | IFI35 | interferon-induced protein 35 |
| 222154_s_at | NM_015535 | DPTP6 | D polymerase-transactivated protein 6 |
| 219356_s_at | NM_016410 | CHMP5 | chromatin modifying protein 5 |
| 219352_at | NM_001013000 | HERC6 | hect domain and RLD 6 |
| 218543_s_at | NM_022750 | PARP12 | poly (ADP-ribose) polymerase family, member 12 |
| 228607_at | NM_001032731 | OAS2 | 2'-5'-oligoadenylate synthetase 2, 69/71kDa |
| 226757_at | NM_001547 | IFIT2 | interferon-induced protein with tetratricopeptide repeats |
| 202446_s_at | NM_021105 | PLSCR1 | phospholipid scramblase 1 |
| 219684_at | NM_022147 | TMEM7 | transmembrane protein 7 |
| 232222_at | NM_017742 | ZCCHC2 | zinc finger, CCHC domain containing 2 |
| 208087_s_at | NM_030776 | ZBP1 | Z-D binding protein 1 |
| 229450_at | NM_001031683 | IFTT3 | interferon-induced protein with tetratricopeptide repeats |
| 225291_at | NM_033109 | PNPT1 | polyribonucleotide nucleotidyltransferase 1 |
| 202086_at | NM_002462 | MX1 | myxovirus resistance 1, interferon-inducible protein p78 |
| 235276_at | NM_001002264 | EPSTI1 | epithelial stromal interaction 1 (breast) |
| 219209_at | NM_022168 | IFIH1 | interferon induced with helicase C domain 1 |
| 209593_s_at | NM_014506 | TOR1B | torsin family 1, member B (torsin B) |
| 228230_at | NM_033405 | PPARAIC285 | peroxisomal proliferator-activated receptor A complex 2 |
| 218986_s_at | NM_017631 | FLJ20035 | hypothetical protein FLJ20035 |
| 228531_at | NM_017654 | SAMD9 | sterile alpha motif domain containing 9 |
| 202869_at | NM_001032409 | OAS1 | 2',5'-oligoadenylate synthetase 1, 40/46kDa |
| 212657_s_at | NM_000577 | IL1RN | interleukin 1 receptor antagonist |
| 202687_s_at | NM_003810 | TNFSF10 | tumor necrosis factor (ligand) superfamily, member 10 |
| 239979_at | NM_001002264 | EPSTI1 | epithelial stromal interaction 1 (breast) |
| 242020_s_at | NM_030776 | ZBP1 | Z-D binding protein 1 |
| 222793_at | NM_014314 | DDX58 | DEAD (Asp-Glu-Ala-Asp) box polypeptide 58 |
| 227807_at | NM_031458 | PARP9 | poly (ADP-ribose) polymerase family, member 9 |
| 200986_at | NM_000062 | SERPING1 | serine (or cysteine) proteinase inhibitor, clade G, member |
| 223501_at | NM_006573 | TNFSF13B | tumor necrosis factor (ligand) superfamily, member 13b |
| 223502_s_at | NM_006573 | TNFSF13B | tumor necrosis factor (ligand) superfamily, member 13b |
| 217502_at | NM_001547 | FIT2 | interferon-induced protein with tetratricopeptide repeats |
| 204994_at | NM_002463 | MX2 | myxovirus resistance 2 |
| 202863_at | NM_003113 | HMG1L3 | high-mobility group protein 1-like 3 |
| 228439_at | NM_138456 | BATF2 | basic leucine zipper transcription factor, AT'F-like 2 |
| 218085_at | NM_016410 | CHMP5 | chromatin modifying protein 5 |
| 219691_at | NM_017654 | SAMD9 | sterile alpha motif domain containing 9 |
| 44673_at | NM_023068 | SN | sialoadhesin |
| 219519_s_at | NM_023068 | SN | sialoadhesin |
| 206133_at | NM_017523 | XIAPAF1 | XIAP associated factor-1, transcript variant 1 |
| 202430_s_at | NM_021105 | PLSCR1 | phospholipid scramblase 1 |
| 243271_at | NM_152703 | SAMD9L | sterile alpha motif domain containing 9-like |
| 205098_at | NM_001295 | CCR1 | chemokine (C-C motif) receptor 1 |
| 231577_s_at | NM_002053 | GBP1 | guanylate binding protein 1, interferon-inducible, 67kDa |
| 202269_x_at | NM_002053 | GBP1 | guanylate binding protein 1, interferon-inducible, 67kDa |
| 241916_at | NM_021105 | PLSCR1 | phospholipid scramblase 1 |
| 205099_s_at | NM_001295 | CCR1 | chemokine (C-C motif) receptor 1 |
| 202270_at | NM_002053 | GBP1 | guanylate binding protein 1, interferon-inducible, 67kDa |

**Table 4. Unique database identifiers and symbols corresponding to unique genes within the list of genes in Table 3.**

| Database Accession | Symbol |
|---|---|
| NM_2080657 | RSAD2 |
| NM_207315 | LOC129607 |
| NM_006417 | IFI44 |
| NM_001002264 | EPSTT1 |
| NM_152703 | SAMD9L |
| NM_006187 | OAS3 |
| NM_016323 | HBRC5 |
| NM_006820 | IFI44L |
| NM_017523 | XIAPAF1 |
| NM_012420 | IFIT5 |
| NM_001032731 | OAS2 |
| NM_005101 | GIP2 |
| NM_017414 | USP18 |
| NM_031458 | PARP9 |
| NM_003733 | OASL |
| NM_001031683 | IFIT3 |
| NM_014314 | DDX58 |
| NM_001001887 | IFIT1 |
| NM_001032409 | OAS1 |
| NM_017554 | PARP14 |
| NM_001572 | IRF7 |
| NM_017742 | ZCCHC2 |
| NM_002346 | LY6E |
| NM_005533 | IFI35 |
| NM_015535 | DPTP6 |
| NM_016410 | CHMP5 |
| NM_001013000 | HERC6 |
| NM_022750 | PARP12 |
| NM_001547 | IFIT2 |
| NM_021105 | PLSCR1 |
| NM_022147 | TMEM7 |
| NM_030776 | ZBP1 |
| NM_033109 | PNPT1 |
| NM_002462 | MX1 |
| NM_022168 | IFIH1 |
| NM_014506 | TOR1B |
| NM_033405 | PPARAIC285 |
| NM_017631 | FLJ20035 |
| NM_017654 | SAMD9 |
| NM_000577 | IL1RN |
| NM_003810 | TNFSF10 |
| NM_000062 | SERPING1 |
| NM_006573 | TNFSF13B |
| NM_002463 | MX2 |
| NM_003113 | HMG1L3 |
| NM_138456 | BATF2 |
| NM_023068 | SN |
| NM_001295 | CCR1 |
| NM_002053 | GBP1 |

**Table 5. Unique database identifiers and symbols corresponding to the list of genes in Table 4 but with genes from Table 1 removed.**

| Database Accession | Symbol |
|---|---|
| NM_017523 | XIAPAP1 |
| NM_001032731 | OAS2 |
| NM_017414 | USP18 |
| NM_003733 | OASL |
| NM_001032409 | OAS1 |
| NM_017554 | PARP14 |
| NM_017742 | ZCCHC2 |
| NM_002346 | LY6E |
| NM_005533 | IFI35 |
| NM_015535 | DPTP6 |
| NM_001013000 | HERC6 |
| NM_022750 | PARP12 |
| NM_001547 | IFIT2 |
| NM_022147 | TMEM7 |
| NM_030776 | ZBP1 |
| NM_033109 | PNPT1 |
| NM_022168 | IFIH1 |
| NM_014506 | TOR1B |
| NM_033405 | PPARAIC285 |
| NM_017631 | FLJ20035 |
| NM_017654 | SAMD9 |
| NM_000577 | IL1RN |
| NM_003810 | TNFSF10 |
| NM_000062 | SERPING1 |
| NM_006573 | TNFSF13B |
| NM_002463 | MX2 |
| NM_003113 | HMG1L3 |
| NM_138456 | BATF2 |
| NM_023068 | SN |
| NM_001295 | CCR1 |
| NM_002053 | GBP1 |

**Table 6. Unique database identifiers and names of combination of genes from the preceding tables.**

| Database Accession | Name |
|---|---|
| NM_207315 (THY1) | similar to thymidylate kinase family LPS-inducible member |
| NM_031458 (PARP9) | B agressive lymphoma gene |
| NM_016323 (HERC5) | cyclin-E binding protein 1 (LOC51191) |
| NM_080657 (RSAD2) | Cig5 |
| NM_004029 (IRF7) | Interferon regulatory factor 7 (IRF7) |
| NM_001S49 (IFTT3) | Interferon induced tetratricopeptide protein IFI60 (IFIT4) |
| NM_002462 (MX1) | Myxovirus influenza resistance 1 |
| BG283489 (PRKR) | FLJ38348 |
| NM_001002264 (EPSTI1) | Putative breast epithelial stromal interaction protein |
| NM_005101 (G1P2) | Interferon-stimulated protein (15 kDa) |
| NM_014314 (DDX58) | AF038963 |
| NM_021105 (PLSCR1) | Phospholipid scramblase P37 |
| NM_006417 (IFI44) | "Interferon-induced, hepatitis C-associated microtubular agg |
| NM_016410 (SNF7DC2) | Chromatin modifying protein 5 |
| NM_012420 (IFIT5) | Retinoic acid- and interferon-inducible protein (58kD) |
| NM_006820 (IFI44L) | chromosome 1 open reading frame 29 |
| NM_006187 (OAS3) | 2'-5'-oligoadenylate synthetase 3 |
| NM_004509(SP110) | Transcriptional coactivator Sp110 |
| NM_152703 (C7orf6) | sterile alpha motif domain containing 9-like |
| NM_005531 (IFI16) | clone MGC:23885 IMAGE:4703266, mRNA, complete cds |
| NM_001001887 (IF1T1) | Interferon-induced protein with tetratricopeptide repeats 1 |
| NM_017523 | XIAP associated factor-1, transcript variant 1 |
| NM_001032731 | 2'-5'-oligoadenylate synthetase 2, 69/711:Da |
| NM_017414 | ubiquitin specific protease 18 |
| NM_003733 | 2'-5'-oligoadenylate synthetase-like |
| NM_001032409 | 2',5'-oligoadenylate synthetase 1, 40/46kDa |
| NM_017554 | poly (ADP-ribose) polymerase family, member 14 |
| NM_017742 | zinc finger, CCHC domain containing 2 |
| NM_002346 | lymphocyte antigen 6 complex, locus E |
| NM_005533 | interferon-induced protein 35 |
| NM_015535 | D polymerase-transactivated protein 6 |
| NM_001013000 | hect domain and RLD 6 |
| NM_022750 | poly (ADP-ribose) polymerase family, member 12 |
| NM_001547 | interferon-induced protein with tetratricopeptide repeats 2 |
| NM_022147 | transmembrane protein 7 |
| NM_030776 | Z-D binding protein 1 |
| NM_033109 | polyribonucleotide nucleotidyltransferase 1 |
| NM_022168 | interferon induced with helicase C domain 1 |
| NM_014506 | torsin family 1, member B (torsin B) |
| NM_033405 | peroxisomal proliferator-activated receptor A complex 285 |
| NM_017631 | hypothetical protein FLJ20035 |
| NM_017654 | sterile alpha motif domain containing 9 |
| NM_000577 | interleukin 1 receptor antagonist |
| NM_003810 | tumor necrosis factor (ligand) superfamily, member 10 |
| NM_000062 | serine (or cysteine) proteinase inhibitor, clade G, member 1 |
| NM_006573 | tumor necrosis factor (ligand) superfamily, member 13b |
| NM_002463 | myxovirus resistance 2 |
| NM_003113 | high-mobility group protein 1-Iike 3 |
| NM_138456 | basic leucine zipper transcription factor, ATF-like 2 |
| NM_023068 | sialoadhesin |
| NM_001295 | chemokine (C-C motif) receptor 1 |
| NM_002053 | guanylate binding protein 1, interferon-inducible, 67kDa |

**Table 7(i) Subset of probesets of Table 3.**

| |
|---|
| 214453_s_at |
| 204972_at |

**Table 7(ii) Subset of probesets of Table 3.**

| |
|---|
| 213797_at |
| 226702_at |
| 214453_s_at |
| 227609_at |
| 242625_at |
| 230036_at |
| 214059_at |
| 218400_at |
| 204972_at |

**Table 7(iii) Subset of probesets of Table 3.**

| |
|---|
| 213797_at |
| 226702_at |
| 214453_s_at |
| 227609_at |
| 242625_at |
| 230036_at |
| 214059_at |
| 218400_at |
| 226603_at |
| 219863_at |
| 204439_at |
| 228617_at |
| 203596_s_at |
| 204972_at |
| 205483_s_at |
| 219211_at |
| 223220_s_at |
| 205660_at |
| 204747_at |
| 218943_s_at |
| 203153_at |
| 205552_s_at |
| 224701_at |
| 208436_s_at |

## Claims

1. A method for diagnosing systemic lupus erythematosus in a subject, said method comprising determining whether the subject comprises a cell that expresses SAMD9L and at least one other gene (or gene associated with probeset) listed in Table 1, 2, 3, 4, 5, 6 or 7 at a level greater than the expression level of the respective genes in a normal reference sample, wherein detection of said cell indicates that the subject has systemic lupus erythematosus.

2. A method of predicting responsiveness of a subject to systemic lupus erythematosus therapy, said method comprising determining whether the subject comprises a cell that expresses SAMD9L and at least one other gene (or gene associated with probeset) listed in Table 1, 2, 3, 4, 5, 6 or 7 at a level greater than the expression level of the respective genes in a normal reference sample, wherein presence of said cell indicates that the subject would be responsive to the systemic lupus erythematosus therapy.

3. A method for assessing predisposition of a subject to develop systemic lupus erythematosus, said method comprising determining whether the subject comprises a cell that expresses SAMD9L and at least one other gene (or gene associated with probeset) listed in Table 1, 2, 3, 4, 5, 6 or 7 at a level greater than the expression level of the respective genes in a normal reference sample, wherein detection of said cell is indicative of a predisposition for the subject to develop systemic lupus erythematosus.

4. The method according to any one of claims 1, 2 or 3 comprising use of an array/gene chip/gene set comprising polynucleotides capable of specifically hybridizing to SAMD9L, and at least one other gene (or gene associated with probeset) listed in Table 1, 2, 3, 4,5, 6 or 7, wherein the array/gene chip/gene set is useful for detecting systemic lupus erythematosus in a subject.

5. The method of any of claims 1-4, wherein said method comprises determining whether the subject comprises a cell that expresses at least 3 of the genes (or genes associated with probesets) listed in Table 1, 2, 3, 4, 5, 6 or 7.

## Patentansprüche

1. Verfahren zum Diagnostizieren von systemischem Lupus erythematodes bei einem Individuum, wobei das Verfahren die Bestimmung umfasst, ob das Individuum eine Zelle umfasst, die SAMD9L und zumindest ein weiteres, in Tabelle 1, 2, 3, 4, 5, 6 oder 7 aufgezähltes Gen (oder ein mit einem Sondensatz assoziiertes Gen) in einem Ausmaß exprimiert, das größer ist als das Ausmaß der Expression der entsprechenden Gene in einer normalen Vergleichsprobe, wobei die Detektion der Zelle ein Indikator dafür ist, dass das Individuum systemischen Lupus erythematodes aufweist.

2. Verfahren zur Voraussage des Ansprechens eines Individuums auf eine Therapie gegen systemischen Lupus erythematodes, wobei das Verfahren die Bestimmung umfasst, ob das Individuum eine Zelle umfasst, die SAMD9L und zumindest ein weiteres, in Tabelle 1, 2, 3, 4, 5, 6 oder 7 aufgezähltes Gen (oder ein mit einem Sondensatz assoziiertes Gen) in einem Ausmaß exprimiert, das größer ist als das Ausmaß der Expression der entsprechenden Gene in einer normalen Vergleichsprobe, wobei die Gegenwart der Zelle ein Indikator dafür ist, dass das Individuum auf eine Therapie gegen systemischen Lupus erythematodes ansprechen würde.

3. Verfahren zur Bestimmung der Veranlagung eines Individuums, systemischen Lupus erythematodes auszubilden, wobei das Verfahren die Bbestimmung umfasst, ob das Individuum eine Zelle umfasst, die SAMD9L und zumindest ein weiteres, in Tabelle 1, 2, 3, 4, 5, 6 oder 7 aufgezähltes Gen (oder ein mit einem Sondensatz assoziiertes Gen) in einem Ausmaß exprimiert, das größer ist als das Ausmaß der Expression der entsprechenden Gene in einer normalen Vergleichsprobe, wobei die Gegenwart der Zelle ein Indikator für die Veranlagung des Individuums ist, systemischen Lupus erythematodes auszubilden.

4. Verfahren nach einem der Ansprüche 1, 2 und 3, umfassend die Verwendung einer Anordnung/eines Genchips/eines Gensatzes, die/der Polynucleotide, die in der Lage sind, spezifisch an SAMD9L zu hybridisieren, und zumindest ein weiteres, in Tabelle 1, 2, 3, 4, 5, 6 oder 7 aufgezähltes Gen (oder ein mit dem Sondensatz assoziiertes Gen) umfasst, worin die Anordnung/der Genchip/der Gensatz zum Detektieren von systemischem Lupus erythematodes bei einem Individuum zweckdienlich ist.

5. Verfahren nach einem der Ansprüche 1 bis 4, worin das Verfahren die Bestimmung umfasst, ob das Individuum eine Zelle umfasst, die zumindest 3 der in Tabelle 1, 2, 3, 4, 5, 6 oder 7 aufgezählten Gene (oder mit Sondensätzen assoziierten Gene) exprimiert.

## Revendications

1. Procédé pour diagnostiquer le lupus érythémateux disséminé chez un sujet, ledit procédé comprenant l'étape consistant à déterminer si le sujet comprend une cellule qui exprime SAMD9L et au moins un autre gène (ou gène associé à une série de sondes) indiqué sur le tableau 1, 2, 3, 4, 5, 6 ou 7 à un taux supérieur au taux d'expression des gènes respectifs dans un échantillon normal de référence, dans lequel la détection de ladite cellule indique que le sujet est atteint de lupus érythémateux disséminé.

2. Procédé pour prévoir la capacité de réponse d'un sujet au lupus à la thérapie contre le lupus érythémateux disséminé, ledit procédé comprenant l'étape consistant à déterminer si le sujet comprend une cellule qui exprime SAMD9L et au moins un autre gène (ou gène associé à une série de sondes) indiqué sur le tableau 1, 2, 3, 4, 5, 6 ou 7 à un taux supérieur au taux d'expression des gènes respectifs dans un échantillon normal de référence, dans lequel la présence de ladite cellule indique que le sujet serait apte à répondre à la thérapie contre le lupus érythémateux disséminé.

3. Procédé pour déterminer la prédisposition d'un sujet au développement du lupus érythémateux disséminé, ledit procédé comprenant l'étape consistant à déterminer si le sujet comprend une cellule qui exprime SAMD9L et au moins un autre gène (ou gène associé à une série de sondes) indiqué sur le tableau 1, 2, 3, 4, 5, 6 ou 7 à un taux supérieur au taux d'expression des gènes respectifs dans un échantillon normal de référence, dans lequel la détection de ladite cellule indique une prédisposition du sujet au développement du lupus érythémateux disséminé.

4. Procédé suivant l'une quelconque des revendications 1, 2 et 3, comprenant l'utilisation d'un réseau/d'une puce porteuse de gènes/d'une série de gènes comprenant des polynucléotides capables de s'hybrider spécifiquement avec SAMD9L et au moins un autre gène (ou gène associé à une série de sondes) indiqué sur le tableau 1, 2, 3, 4, 5, 6 ou 7, dans lequel le réseau/la puce porteuse de gènes/la série de gènes est utile pour la détection du lupus érythémateux disséminé chez un sujet.

5. Procédé suivant l'une quelconque des revendications 1 à 4, ledit procédé comprenant l'étape consistant à déterminer si le sujet comprend une cellule qui exprime au moins 3 des gènes (ou gènes associés à des séries de sondes) indiqués sur le tableau 1, 2, 3, 4, 5, 6 ou 7.
